# EUROPEAN PATENT APPLICATION

(11) **EP 3 772 514 A1**
(43) Date of publication of application: **10.02.2021**
(21) Application number: 19190760.9
(22) Date of filing: 08.08.2019
(51) Int. Cl.: C07D 405/14, A01N 43/50, A01N 43/76, A01N 43/78, C07D 407/06, C07D 413/14, C07D 417/14, C07D 493/04

(54) **HERBICIDAL AMIDES**

(71) Applicant: BASF SE, 67056 Ludwigshafen am Rhein (DE)
(72) Inventor: Witschel, Matthias, 67056 Ludwigshafen (DE); Geerdink, Danny, 67056 Ludwigshafen (DE); Seitz, Thomas, 67056 Ludwigshafen (DE); Kraemer, Gerd, 67117 Limburgerhof (DE); Newton, Trevor William, 67117 Limburgerhof (DE); Hollenbach, Eva, 67117 Limburgerhof (DE)
(74) Representative: BASF IP Association

(57) **Abstract**

The present invention relates to amides of formula (I) 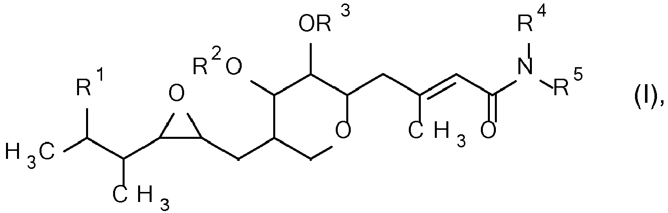 wherein the variables are defined according to the description, process for preparation, their use as herbicides, i.e. for controlling harmful plants, and also a method for controlling unwanted vegetation which comprises allowing a herbicidal effective amount of at least one amide of formula (I) to act on plants, their seed and/or their habitat.

## Description

The present invention relates to amides of formula (I) defined below and to their use as herbicides.

WO 93/19599 describes structurally similar monic amides, for which herbicidal action is stated, which differ from the amides of formula (I) according to the present invention in the amid substituent.

However, the herbicidal properties of these known compounds regarding the undesired vegetation are not always entirely satisfactory.

It is therefore an object of the present invention to provide amides of formula (I) having improved herbicidal action. To be provided are in particular amides of formula (I) which have high herbicidal activity, in particular even at low application rates, and which are sufficiently compatible with crop plants for commercial utilization.

These and further objects are achieved by amides of formula (I), defined below.

Accordingly, the present invention provides amides of formula (I) 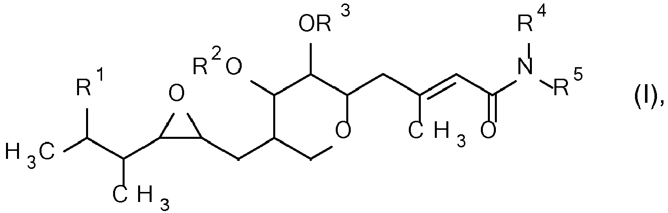 wherein the variables have the following meanings:
- R¹: OH, =O or O(CO)R⁶,
wherein R⁶ is H or C₁-C₆-alkyl;
- R²: H or (CO)R⁷; wherein R⁷ is H or C₁-C₆-alkyl;
- R³: H or (CO)R⁸;
wherein R⁸ is H or C₁-C₆-alkyl; or
- R² and R³: together form -CR⁹R¹⁰-,
wherein R⁹ and R¹⁰ independently of one another are H, C₁-C₆-alkyl or C₁-C₆-alkoxy;
- R⁴: H;
- R⁵: C₂-C₆-haloalkyl, C₃-C₆-haloalkenyl, C₃-C₆-alkynyl or C₃-C₆-haloalkynyl; or
- R⁴ and R⁵: together form a 5- to 6-membered saturated heterocycle,
which is substituted with one carbonyl group;
optionally has in addition to the N-atom one ring member selected from the group consisting of -N-, -O- and -S-, and
optionally is substituted with one or two substituents selected from C₁-C₃-alkyl and C₁-C₃-haloalkyl.

The present invention also provides formulations comprising at least one amides of formula (I) and auxiliaries customary for formulating crop protection agents.

The present invention also provides the use of amides of formula (I) as herbicides, i.e. for controlling undesired vegetation.

The present invention furthermore provides a method for controlling undesired vegetation where a herbicidal effective amount of at least one amide of formula (I) is allowed to act on plants, their seeds and/or their habitat.

Moreover, the invention relates to processes for preparing amides of formula (I).

The organic moieties mentioned in the definition of the variables R¹ to R¹⁰, are - like the term halogen - collective terms for individual enumerations of the individual group members. The term halogen denotes in each case fluorine, chlorine, bromine or iodine. All hydrocarbon chains can be straight-chain or branched, the prefix Cₙ-Cₘ denoting in each case the possible number of carbon atoms in the group.

Examples of such meanings are:
- C₁-C₃-alkyl: CH₃, C₂H₅, n-propyl, and CH(CH₃)₂;
- C₁-C₄-alkyl: for example CH₃, C₂H₅, n-propyl, CH(CH₃)₂, n-butyl, CH(CH₃)-C₂H₅, CH₂-CH(CH₃)₂ and C(CH₃)₃;
- C₂-C₄-alkyl: for example C₂H₅, n-propyl, CH(CH₃)₂, n-butyl, CH(CH₃)-C₂H₅, CH₂-CH(CH₃)₂ and C(CH₃)₃;
- C₁-C₆-alkyl: C₁-C₄-alkyl as mentioned above, and also, for example, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl or 1-ethyl-2-methylpropyl, preferably methyl, ethyl, n-propyl, 1-methylethyl, n-butyl, 1,1-dimethylethyl, n-pentyl or n-hexyl;
- C₁-C₃-haloalkyl: C₁-C₃-alkyl as mentioned above which is partially or fully substituted by fluorine, chlorine, bromine and/or iodine, i.e., for example, chloromethyl, dichloromethyl, trichloromethyl, fluoromethyl, difluoromethyl, trifluoromethyl, chlorofluoromethyl, dichlorofluoromethyl, chlorodifluoromethyl, bromomethyl, iodomethyl, 2-fluoroethyl, 2-chloroethyl, 2-bromoethyl, 2-iodoethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, 2,2,3,3,3-pentafluoropropyl, heptafluoropropyl;
- C₂-C₄-haloalkyl: C₂-C₄-alkyl as mentioned above which is partially or fully substituted by fluorine, chlorine, bromine and/or iodine, for example 2-fluoroethyl, 2-chloroethyl, 2-bromoethyl, 2-iodoethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, 2,2,3,3,3-pentafluoropropyl, heptafluoropropyl, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl, nonafluorobutyl, 1,1,2,2,-tetrafluoroethyl and 1-trifluoromethyl-1,2,2,2-tetrafluoroethyl;
- C₂-C₆-haloalkyl: C₂-C₄-haloalkyl as mentioned above, and also, for example, 5-fluoropentyl, 5-chloropentyl, 5-bromopentyl, 5-iodopentyl, undecafluoropentyl, 6-fluorohexyl, 6-chlorohexyl, 6-bromohexyl, 6-iodohexyl and dodecafluorohexyl;
- C₃-C₆-alkenyl: for example 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl;
- C₃-C₆-haloalkenyl: a C₃-C₆-alkenyl radical as mentioned above which is partially or fully substituted by fluorine, chlorine, bromine and/or iodine, for example 2-fluoroprop-2-en-1-yl, 3-fluoroprop-2-en-1-yl, 2,3-difluoroprop-2-en-1-yl, 3,3-difluoroprop-2-en-1-yl, 2,3,3-trifluoro-2-en-1-yl, 2,3-difluorobut-2-en-1-yl, 2-chloroprop-2-en-1-yl, 3-chloroprop-2-en-1-yl, 2,3-dichloroprop-2-en-1-yl, 3,3-dichloroprop-2-en-1-yl, 2,3,3-trichloro-2-en-1-yl, 2,3-dichlorobut-2-en-1-yl, 2-bromoprop-2-en-1-yl, 3-bromoprop-2-en-1-yl, 2,3-dibromoprop-2-en-1-yl, 3,3-dibromoprop-2-en-1-yl, 2,3,3-tribromo-2-en-1-yl or 2,3-dibromobut-2-en-1-yl;
- C₃-C₆-alkynyl: for example 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl-2-propynyl, 1-hexynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl and 1-ethyl-1-methyl-2-propynyl;
- C₃-C₆-haloalkynyl: a C₃-C₆-alkynyl radical as mentioned above which is partially or fully substituted by fluorine, chlorine, bromine and/or iodine, for example 1,1-difluoroprop-2-yn-1-yl, 3-chloroprop-2-yn-1-yl, 3-bromoprop-2-yn-1-yl, 3-iodoprop-2-yn-1-yl, 4-fluorobut-2-yn-1-yl, 4-chlorobut-2-yn-1-yl, 1,1-difluorobut-2-yn-1-yl, 4-iodobut-3-yn-1-yl, 5-fluoropent-3-yn-1-yl, 5-iodopent-4-yn-1-yl, 6-fluorohex-4-yn-1-yl or 6-iodohex-5-yn-1-yl;
- C₁-C₄-alkoxy: for example, methoxy, ethoxy, propoxy, 1-methylethoxy butoxy, 1-methylpropoxy, 2-methylpropoxy and 1,1-dimethylethoxy;
- C₁-C₆-alkoxy: C₁-C₄-alkoxy as mentioned above, and also, for example, pentoxy, 1-methylbutoxy, 2-methylbutoxy, 3-methoxylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy and 1-ethyl-2-methylpropoxy.

The preferred embodiments of the invention mentioned herein below have to be understood as being preferred either independently from each other or in combination with one another.

According to a preferred embodiment of the invention preference is also given to those amides of formula (I), wherein the variables, either independently of one another or in combination with one another, have the following meanings:
Preferred are the amides of formula (I), wherein
- R¹: is OH, =O, O(CO)H or O(CO)CH₃; particularly preferred OH or =O; especially preferred OH; also especially preferred =O.

Also preferred are the amides of formula (I), wherein
- R²: is H, (CO)H or (CO)CH₃; particularly preferred H or (CO)H; especially preferred H; also especially preferred (CO)H.

Also preferred are the amides of formula (I), wherein
- R³: is H, (CO)H or (CO)CH₃; particularly preferred H or (CO)H; especially preferred H; also especially preferred (CO)H.

Also preferred are the amides of formula (I), wherein R² and R³ have the same meaning.

Also preferred are the amides of formula (I), wherein
R² and R³ together form -CR⁹R¹⁰-, wherein
- R⁹: is C₁-C₆-alkyl or C₁-C₆-alkoxy; particularly preferred C₁-C₄-alkyl or C₁-C₄-alkoxy especially preferred CH₃, OCH₃ or OCH₂CH₃, and
- R¹⁰: is H or C₁-C₆-alkyl; particularly preferred H or C₁-C₄-alkyl; especially preferred H or CH₃.

Also preferred are the amides of formula (I), wherein
- R⁵: is C₂-C₆-haloalkyl, C₃-C₆-haloalkenyl or C₃-C₆-alkynyl;
particularly preferred C₂-C₄-haloalkyl, C₃-C₆-haloalkenyl or C₃-C₆-alkynyl;
especially preferred C₂-C₄-haloalkyl or C₃-C₆-alkynyl more preferred C₂-haloalkyl or propargyl.

Also preferred are the amides of formula (I), wherein
- R⁴ and R⁵: together form a 5-membered saturated heterocycle,
which is substituted with one carbonyl group, optionally has in addition to the N-atom one ring member selected from the group consisting of -N-, -O- and -S-, and
optionally is substituted with one or two substituents selected from C₁-C₃-alkyl and C₁-C₃-haloalkyl;
particularly preferred together form a 5-membered saturated heterocycle, which is substituted with one carbonyl group, optionally has in addition to the N-atom one ring member selected from -O- and optionally is substituted with one or two substituents selected from C₁-C₃-alkyl and C₁-C₃-haloalkyl;
especially preferred together form a 5-membered saturated heterocycle, which is substituted with one carbonyl group, has in addition to the N-atom one ring member selected from -O, and optionally is substituted with one or two substituents selected from C₁-C₃-alkyl and C₁-C₃-haloalkyl.

Also preferred are the amides of formula (I), wherein
- R⁴ and R⁵: together form a 5-membered saturated heterocycle selected from the group consisting of A-1 to A-3: 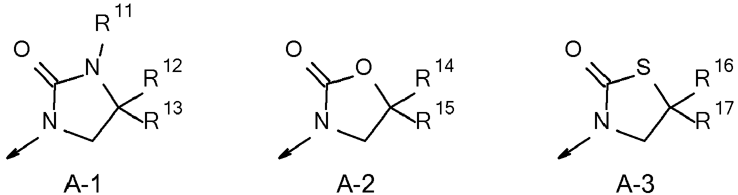
wherein R¹¹ to R¹⁷ have the following meanings:
- R¹¹: H, C₁-C₃-alkyl or C₁-C₃-haloalkyl; preferably C₁-C₃-alkyl or C₁-C₃-haloalkyl; especially preferred CH₃, CF₃ or CH₂CF₃; more preferred CH₃ or CH₂CF₃;
- R¹²: H, C₁-C₃-alkyl or C₁-C₃-haloalkyl; preferably H, CH₃ or CF₃; especially preferred H or CH₃; more preferred H;
- R¹³: H, C₁-C₃-alkyl or C₁-C₃-haloalkyl; preferably H, CH₃ or CF₃; especially preferred H or CH₃; more preferred H;
- R¹⁴: H, C₁-C₃-alkyl or C₁-C₃-haloalkyl; preferably H, CH₃ or CF₃; especially preferred H; also especially preferred CH₃; also especially preferred CF₃;
- R¹⁵: H, C₁-C₃-alkyl or C₁-C₃-haloalkyl; preferably H, CH₃ or CF₃; especially preferred H or CH₃; more preferred H; also more preferred CH₃;
- R¹⁶: H, C₁-C₃-alkyl or C₁-C₃-haloalkyl; preferably H, CH₃ or CF₃; especially preferred H;
- R¹⁷: H, C₁-C₃-alkyl or C₁-C₃-haloalkyl; preferably H, CH₃ or CF₃; especially preferred H.
particularly preferred together form a 5-membered saturated heterocycle selected from the group consisting of A-1 and A-2,
wherein R¹¹ to R¹⁵ are as defined above;
especially preferred together form a 5-membered saturated heterocycle selected from A-2, wherein R¹⁴ and R¹⁵ are as defined above.

Also preferred are the amides of formula (I), wherein
- R⁶: is H or CH₃;
particularly preferred H;
also particularly preferred CH₃.

Also preferred are the amides of formula (I), wherein
- R⁷ is: H or CH₃;
particularly preferred H;
also particularly preferred CH₃.

Also preferred are the amides of formula (I), wherein
- R⁸ is: H or CH₃;
particularly preferred H;
also particularly preferred CH₃.

Also preferred are the amides of formula (I), wherein
R¹ is OH, =O, O(CO)H or O(CO)CH₃;
R² is H, (CO)H or (CO)CH₃;
R³ is H, (CO)H or (CO)CH₃;
R⁵ is C₂-C₆-haloalkyl, C₃-C₆-haloalkenyl or C₃-C₆-alkynyl; or
- R⁴ and R⁵: together form a 5-membered saturated heterocycle,
which is substituted with one carbonyl group,
optionally has in addition to the N-atom one ring member selected from the group consisting of -N-, -O- and -S-, and
optionally is substituted with one or two substituents selected from C₁-C₃-alkyl and C₁-C₃-haloalkyl;
particularly preferred are the amides of formula (I), wherein
R¹ is OH or =O;
R² is H or (CO)H;
R³ is H or (CO)H;
R⁵ is C₂-C₄-haloalkyl or C₃-C₆-alkynyl; or
- R⁴ and R⁵: together form a 5-membered saturated heterocycle selected from the group consisting of A-1 to A-3: 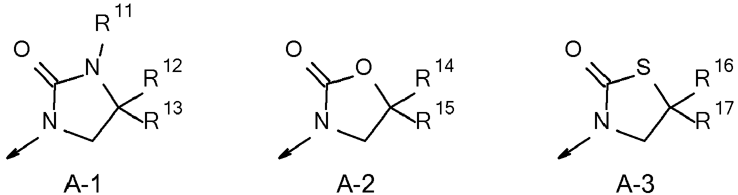
wherein R¹¹ to R¹⁷ have the following meanings:
R¹¹ C₁-C₃-alkyl or C₁-C₃-haloalkyl;
R¹², R¹³, R¹⁴, R¹⁵, R¹⁶, R¹⁷ independently of one another H, C₁-C₃-alkyl or C₁-C₃-haloalkyl;
especially preferred are the amides of formula (I), wherein
R¹ is OH or =O;
R² is H;
R³ is H;
R⁵ is C₂-haloalkyl or propargyl, or
- R⁴ and R⁵: together form a 5-membered saturated heterocycle selected from the group consisting of A-2: 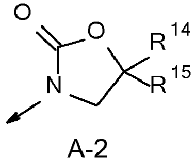
wherein R¹⁴ and R¹⁵ independently of one another are H, C₁-C₃-alkyl or C₁-C₃-haloalkyl.

Particular preference is given to amides of formula (I.a) (corresponds to formula (I) wherein R¹ is OH): 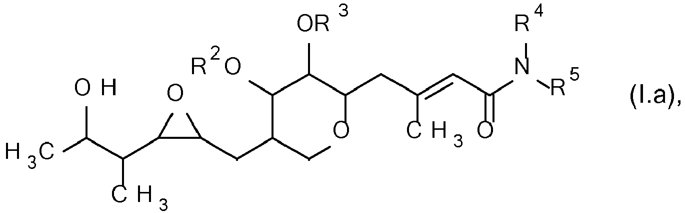 wherein the variables R², R³, R⁴ and R⁵ have the meanings, in particular the preferred meanings, as defined above.

Special preference is given to the amides of the formulae (I.a.1) to (I.a.208) of Table A, where the definitions of the variables R², R³, R⁴ and R⁵ are of particular importance for the compounds according to the invention not only in combination with one another but in each case also on their own:

**Table A**

| No. | R² | R³ | R⁴ | R⁵ |
|---|---|---|---|---|
| I.a.1 | H | H | H | CH₂CH₂F |
| I.a.2 | H | H | H | CH₂CHF₂ |
| I.a.3 | H | H | H | CH₂CF₃ |
| I.a.4 | H | H | H | CH₂CF₂Br |
| I.a.5 | H | H | H | CH₂CH₂CHF₂ |
| I.a.6 | H | H | H | CH₂CH₂CF₃ |
| I.a.7 | H | H | H | CH₂CF₂CH₃ |
| I.a.8 | H | H | H | CH₂CF₂CHF₂ |
| I.a.9 | H | H | H | CH₂CF₂CF₃ |
| I.a.10 | H | H | H | CH₂CF₂CF₂CF₃ |
| I.a.11 | H | H | H | (*S*)-CH(CH₃)(CF₃) |
| I.a.12 | H | H | H | CH₂CH=CCl₂ |
| I.a.13 | H | H | H | CH₂CF=CH₂ |
| I.a.14 | H | H | H | CH₂CCl=CH₂ |
| I.a.15 | H | H | H | CH₂CBr=CH₂ |
| I.a.16 | H | H | H | CH₂C≡CH |
| I.a.17 | H | H | H | CH₂C≡CCH₃ |
| I.a.18 | H | H | H | CH(CH₃)C≡CH |
| I.a.19 | H | H | H | CH₂CH₂C≡CH |
| I.a.20 | H | H | H | CH₂C≡CC(CH₃)₃ |
| I.a.21 | H | H | -(CO)OCH₂CH₂- | |
| I.a.22 | H | H | -(CO)SCH₂CH₂- | |
| I.a.23 | H | H | -(CO)OC(CH₃)₂CH₂- | |
| I.a.24 | H | H | -(CO)OCH(CF₃)CH₂- | |
| I.a.25 | H | H | -(CO)N(CH₃)CH₂CH₂- | |
| I.a.26 | H | H | -(CO)N(CH₂CF₃)CH₂CH₂- | |
| I.a.27 | H | (CO)H | H | CH₂CH₂F |
| I.a.28 | H | (CO)H | H | CH₂CHF₂ |
| I.a.29 | H | (CO)H | H | CH₂CF₃ |
| I.a.30 | H | (CO)H | H | CH₂CF₂Br |
| I.a.31 | H | (CO)H | H | CH₂CH₂CHF₂ |
| I.a.32 | H | (CO)H | H | CH₂CH₂CF₃ |
| I.a.33 | H | (CO)H | H | CH₂CF₂CH₃ |
| I.a.34 | H | (CO)H | H | CH₂CF₂CHF₂ |
| I.a.35 | H | (CO)H | H | CH₂CF₂CF₃ |
| I.a.36 | H | (CO)H | H | CH₂CF₂CF₂CF₃ |
| I.a.37 | H | (CO)H | H | (S)-CH(CH₃)(CF₃) |
| I.a.38 | H | (CO)H | H | CH₂CH=CCl₂ |
| I.a.39 | H | (CO)H | H | CH₂CF=CH₂ |
| I.a.40 | H | (CO)H | H | CH₂CCl=CH₂ |
| I.a.41 | H | (CO)H | H | CH₂CBr=CH₂ |
| I.a.42 | H | (CO)H | H | CH₂C≡CH |
| I.a.43 | H | (CO)H | H | CH₂C≡CCH₃ |
| I.a.44 | H | (CO)H | H | CH(CH₃)C≡CH |
| I.a.45 | H | (CO)H | H | CH₂CH₂C≡CH |
| I.a.46 | H | (CO)H | H | CH₂C≡CC(CH₃)₃ |
| I.a.47 | H | (CO)H | -(CO)OCH₂CH₂- | |
| I.a.48 | H | (CO)H | -(CO)SCH₂CH₂- | |
| I.a.49 | H | (CO)H | -(CO)OC(CH₃)₂CH₂- | |
| I.a.50 | H | (CO)H | -(CO)OCH(CF₃)CH₂- | |
| I.a.51 | H | (CO)H | -(CO)N(CH₃)CH₂CH₂- | |
| I.a.52 | H | (CO)H | -(CO)N(CH₂CF₃)CH₂CH₂- | |
| I.a.53 | (CO)H | H | H | CH₂CH₂F |
| I.a.54 | (CO)H | H | H | CH₂CHF₂ |
| I.a.55 | (CO)H | H | H | CH₂CF₃ |
| I.a.56 | (CO)H | H | H | CH₂CF₂Br |
| I.a.57 | (CO)H | H | H | CH₂CH₂CHF₂ |
| I.a.58 | (CO)H | H | H | CH₂CH₂CF₃ |
| I.a.59 | (CO)H | H | H | CH₂CF₂CH₃ |
| I.a.60 | (CO)H | H | H | CH₂CF₂CHF₂ |
| I.a.61 | (CO)H | H | H | CH₂CF₂CF₃ |
| I.a.62 | (CO)H | H | H | CH₂CF₂CF₂CF₃ |
| I.a.63 | (CO)H | H | H | (S)-CH(CH₃)(CF₃) |
| I.a.64 | (CO)H | H | H | CH₂CH=CCl₂ |
| I.a.65 | (CO)H | H | H | CH₂CF=CH₂ |
| I.a.66 | (CO)H | H | H | CH₂CCl=CH₂ |
| I.a.67 | (CO)H | H | H | CH₂CBr=CH₂ |
| I.a.68 | (CO)H | H | H | CH₂C≡CH |
| I.a.69 | (CO)H | H | H | CH₂C≡CCH₃ |
| I.a.70 | (CO)H | H | H | CH(CH₃)C≡CH |
| I.a.71 | (CO)H | H | H | CH₂CH₂C≡CH |
| I.a.72 | (CO)H | H | H | CH₂C≡CC(CH₃)₃ |
| I.a.73 | (CO)H | H | -(CO)OCH₂CH₂- | |
| I.a.74 | (CO)H | H | -(CO)SCH₂CH₂- | |
| I.a.75 | (CO)H | H | -(CO)OC(CH₃)₂CH₂- | |
| I.a.76 | (CO)H | H | -(CO)OCH(CF₃)CH₂- | |
| I.a.77 | (CO)H | H | -(CO)N(CH₃)CH₂CH₂- | |
| I.a.78 | (CO)H | H | -(CO)N(CH₂CF₃)CH₂CH₂- | |
| I.a.79 | (CO)H | (CO)H | H | CH₂CH₂F |
| I.a.80 | (CO)H | (CO)H | H | CH₂CHF₂ |
| I.a.81 | (CO)H | (CO)H | H | CH₂CF₃ |
| I.a.82 | (CO)H | (CO)H | H | CH₂CF₂Br |
| I.a.83 | (CO)H | (CO)H | H | CH₂CH₂CHF₂ |
| I.a.84 | (CO)H | (CO)H | H | CH₂CH₂CF₃ |
| I.a.85 | (CO)H | (CO)H | H | CH₂CF₂CH₃ |
| I.a.86 | (CO)H | (CO)H | H | CH₂CF₂CHF₂ |
| I.a.87 | (CO)H | (CO)H | H | CH₂CF₂CF₃ |
| I.a.88 | (CO)H | (CO)H | H | CH₂CF₂CF₂CF₃ |
| I.a.89 | (CO)H | (CO)H | H | (S)-CH(CH₃)(CF₃) |
| I.a.90 | (CO)H | (CO)H | H | CH₂CH=CCl₂ |
| I.a.91 | (CO)H | (CO)H | H | CH₂CF=CH₂ |
| I.a.92 | (CO)H | (CO)H | H | CH₂CCl=CH₂ |
| I.a.93 | (CO)H | (CO)H | H | CH₂CBr=CH₂ |
| I.a.94 | (CO)H | (CO)H | H | CH₂C≡CH |
| I.a.95 | (CO)H | (CO)H | H | CH₂C≡CCH₃ |
| I.a.96 | (CO)H | (CO)H | H | CH(CH₃)C≡CH |
| I.a.97 | (CO)H | (CO)H | H | CH₂CH₂C≡CH |
| I.a.98 | (CO)H | (CO)H | H | CH₂C≡CC(CH₃)₃ |
| I.a.99 | (CO)H | (CO)H | -(CO)OCH₂CH₂- | |
| I.a.100 | (CO)H | (CO)H | -(CO)SCH₂CH₂- | |
| I.a.101 | (CO)H | (CO)H | -(CO)OC(CH₃)₂CH₂- | |
| I.a.102 | (CO)H | (CO)H | -(CO)OCH(CF₃)CH₂- | |
| I.a.103 | (CO)H | (CO)H | -(CO)N(CH₃)CH₂CH₂- | |
| I.a.104 | (CO)H | (CO)H | -(CO)N(CH₂CF₃)CH₂CH₂- | |
| I.a.105 | (CO)CH₃ | (CO)CH₃ | H | CH₂CH₂F |
| I.a.106 | (CO)CH₃ | (CO)CH₃ | H | CH₂CHF₂ |
| I.a.107 | (CO)CH₃ | (CO)CH₃ | H | CH₂CF₃ |
| I.a.108 | (CO)CH₃ | (CO)CH₃ | H | CH₂CF₂Br |
| I.a.109 | (CO)CH₃ | (CO)CH₃ | H | CH₂CH₂CHF₂ |
| I.a.110 | (CO)CH₃ | (CO)CH₃ | H | CH₂CH₂CF₃ |
| I.a.111 | (CO)CH₃ | (CO)CH₃ | H | CH₂CF₂CH₃ |
| I.a.112 | (CO)CH₃ | (CO)CH₃ | H | CH₂CF₂CHF₂ |
| I.a.113 | (CO)CH₃ | (CO)CH₃ | H | CH₂CF₂CF₃ |
| I.a.114 | (CO)CH₃ | (CO)CH₃ | H | CH₂CF₂CF₂CF₃ |
| I.a.115 | (CO)CH₃ | (CO)CH₃ | H | (S)-CH(CH₃)(CF₃) |
| I.a.116 | (CO)CH₃ | (CO)CH₃ | H | CH₂CH=CCl₂ |
| I.a.117 | (CO)CH₃ | (CO)CH₃ | H | CH₂CF=CH₂ |
| I.a.118 | (CO)CH₃ | (CO)CH₃ | H | CH₂CCl=CH₂ |
| I.a.119 | (CO)CH₃ | (CO)CH₃ | H | CH₂CBr=CH₂ |
| I.a.120 | (CO)CH₃ | (CO)CH₃ | H | CH₂C≡CH |
| I.a.121 | (CO)CH₃ | (CO)CH₃ | H | CH₂C≡CCH₃ |
| I.a.122 | (CO)CH₃ | (CO)CH₃ | H | CH(CH₃)C≡CH |
| I.a.123 | (CO)CH₃ | (CO)CH₃ | H | CH₂CH₂C≡CH |
| I.a.124 | (CO)CH₃ | (CO)CH₃ | H | CH₂C≡CC(CH₃)₃ |
| I.a.125 | (CO)CH₃ | (CO)CH₃ | -(CO)OCH₂CH₂- | |
| I.a.126 | (CO)CH₃ | (CO)CH₃ | -(CO)SCH₂CH₂- | |
| I.a.127 | (CO)CH₃ | (CO)CH₃ | -(CO)OC(CH₃)₂CH₂- | |
| I.a.128 | (CO)CH₃ | (CO)CH₃ | -(CO)OCH(CF₃)CH₂- | |
| I.a.129 | (CO)CH₃ | (CO)CH₃ | -(CO)N(CH₃)CH₂CH₂- | |
| I.a.130 | (CO)CH₃ | (CO)CH₃ | -(CO)N(CH₂CF₃)CH₂CH₂- | |
| I.a.131 | -C(CH₃)₂- | | H | CH₂CH₂F |
| I.a.132 | -C(CH₃)₂- | | H | CH₂CHF₂ |
| I.a.133 | -C(CH₃)₂- | | H | CH₂CF₃ |
| I.a.134 | -C(CH₃)₂- | | H | CH₂CF₂Br |
| I.a.135 | -C(CH₃)₂- | | H | CH₂CH₂CHF₂ |
| I.a.136 | -C(CH₃)₂- | | H | CH₂CH₂CF₃ |
| I.a.137 | -C(CH₃)₂- | | H | CH₂CF₂CH₃ |
| I.a.138 | -C(CH₃)₂- | | H | CH₂CF₂CHF₂ |
| I.a.139 | -C(CH₃)₂- | | H | CH₂CF₂CF₃ |
| I.a.140 | -C(CH₃)₂- | | H | CH₂CF₂CF₂CF₃ |
| I.a.141 | -C(CH₃)₂- | | H | (S)-CH(CH₃)(CF₃) |
| I.a.142 | -C(CH₃)₂- | | H | CH₂CH=CCl₂ |
| I.a.143 | -C(CH₃)₂- | | H | CH₂CF=CH₂ |
| I.a.144 | -C(CH₃)₂- | | H | CH₂CCl=CH₂ |
| I.a.145 | -C(CH₃)₂- | | H | CH₂CBr=CH₂ |
| I.a.146 | -C(CH₃)₂- | | H | CH₂C≡CH |
| I.a.147 | -C(CH₃)₂- | | H | CH₂C≡CCH₃ |
| I.a.148 | -C(CH₃)₂- | | H | CH(CH₃)C≡CH |
| I.a.149 | -C(CH₃)₂- | | H | CH₂CH₂C≡CH |
| I.a.150 | -C(CH₃)₂- | | H | CH₂C≡CC(CH₃)₃ |
| I.a.151 | -C(CH₃)₂- | | -(CO)OCH₂CH₂- | |
| I.a.152 | -C(CH₃)₂- | | -(CO)SCH₂CH₂- | |
| I.a.153 | -C(CH₃)₂- | | -(CO)OC(CH₃)₂CH₂- | |
| I.a.154 | -C(CH₃)₂- | | -(CO)OCH(CF₃)CH₂- | |
| I.a.155 | -C(CH₃)₂- | | -(CO)N(CH₃)CH₂CH₂- | |
| I.a.156 | -C(CH₃)₂- | | -(CO)N(CH₂CF₃)CH₂CH₂- | |
| I.a.157 | -C(C₂H₅)₂- | | H | CH₂CH₂F |
| I.a.158 | -C(C₂H₅)₂- | | H | CH₂CHF₂ |
| I.a.159 | -C(C₂H₅)₂- | | H | CH₂CF₃ |
| I.a.160 | -C(C₂H₅)₂- | | H | CH₂CF₂Br |
| I.a.161 | -C(C₂H₅)₂- | | H | CH₂CH₂CHF₂ |
| I.a.162 | -C(C₂H₅)₂- | | H | CH₂CH₂CF₃ |
| I.a.163 | -C(C₂H₅)₂- | | H | CH₂CF₂CH₃ |
| I.a.164 | -C(C₂H₅)₂- | | H | CH₂CF₂CHF₂ |
| I.a.165 | -C(C₂H₅)₂- | | H | CH₂CF₂CF₃ |
| I.a.166 | -C(C₂H₅)₂- | | H | CH₂CF₂CF₂CF₃ |
| I.a.167 | -C(C₂H₅)₂- | | H | (*S*)-CH(CH₃)(CF₃) |
| I.a.168 | -C(C₂H₅)₂- | | H | CH₂CH=CCl₂ |
| I.a.169 | -C(C₂H₅)₂- | | H | CH₂CF=CH₂ |
| I.a.170 | -C(C₂H₅)₂- | | H | CH₂CCl=CH₂ |
| I.a.171 | -C(C₂H₅)₂- | | H | CH₂CBr=CH₂ |
| I.a.172 | -C(C₂H₅)₂- | | H | CH₂C≡CH |
| I.a.173 | -C(C₂H₅)₂- | | H | CH₂C≡CCH₃ |
| I.a.174 | -C(C₂H₅)₂- | | H | CH(CH₃)C≡CH |
| I.a.175 | -C(C₂H₅)₂- | | H | CH₂CH₂C≡CH |
| I.a.176 | -C(C₂H₅)₂- | | H | CH₂C≡CC(CH₃)₃ |
| I.a.177 | -C(C₂H₅)₂- | | -(CO)OCH₂CH₂- | |
| I.a.178 | -C(C₂H₅)₂- | | -(CO)SCH₂CH₂- | |
| I.a.179 | -C(C₂H₅)₂- | | -(CO)OC(CH₃)₂CH₂- | |
| I.a.180 | -C(C₂H₅)₂- | | -(CO)OCH(CF₃)CH₂- | |
| I.a.181 | -C(C₂H₅)₂- | | -(CO)N(CH₃)CH₂CH₂- | |
| I.a.182 | -C(C₂H₅)₂- | | -(CO)N(CH₂CF₃)CH₂CH₂- | |
| I.a.183 | -CH(OC₂H₅)- | | H | CH₂CH₂F |
| I.a.184 | -CH(OC₂H₅)- | | H | CH₂CHF₂ |
| I.a.185 | -CH(OC₂H₅)- | | H | CH₂CF₃ |
| I.a.186 | -CH(OC₂H₅)- | | H | CH₂CF₂Br |
| I.a.187 | -CH(OC₂H₅)- | | H | CH₂CH₂CHF₂ |
| I.a.188 | -CH(OC₂H₅)- | | H | CH₂CH₂CF₃ |
| I.a.189 | -CH(OC₂H₅)- | | H | CH₂CF₂CH₃ |
| I.a.190 | -CH(OC₂H₅)- | | H | CH₂CF₂CHF₂ |
| I.a.191 | -CH(OC₂H₅)- | | H | CH₂CF₂CF₃ |
| I.a.192 | -CH(OC₂H₅)- | | H | CH₂CF₂CF₂CF₃ |
| I.a.193 | -CH(OC₂H₅)- | | H | (*S*)-CH(CH₃)(CF₃) |
| I.a.194 | -CH(OC₂H₅)- | | H | CH₂CH=CCl₂ |
| I.a.195 | -CH(OC₂H₅)- | | H | CH₂CF=CH₂ |
| I.a.196 | -CH(OC₂H₅)- | | H | CH₂CCl=CH₂ |
| I.a.197 | -CH(OC₂H₅)- | | H | CH₂CBr=CH₂ |
| I.a.198 | -CH(OC₂H₅)- | | H | CH₂C≡CH |
| I.a.199 | -CH(OC₂H₅)- | | H | CH₂C≡CCH₃ |
| I.a.200 | -CH(OC₂H₅)- | | H | CH(CH₃)C≡CH |
| I.a.201 | -CH(OC₂H₅)- | | H | CH₂CH₂C≡CH |
| I.a.202 | -CH(OC₂H₅)- | | H | CH₂C≡CC(CH₃)₃ |
| I.a.203 | -CH(OC₂H₅)- | | -(CO)OCH₂CH₂- | |
| I.a.204 | -CH(OC₂H₅)- | | -(CO)SCH₂CH₂- | |
| I.a.205 | -CH(OC₂H₅)- | | -(CO)OC(CH₃)₂CH₂- | |
| I.a.206 | -CH(OC₂H₅)- | | -(CO)OCH(CF₃)CH₂- | |
| I.a.207 | -CH(OC₂H₅)- | | -(CO)N(CH₃)CH₂CH₂- | |
| I.a.208 | -CH(OC₂H₅)- | | -(CO)N(CH₂CF₃)CH₂CH₂- | |

Also preferred are the amides of formula (I.b), particularly preferred the amides of formulae (I.b.1) to (I.b.208), which differ from the corresponding amides of formulae (I.a.1) to (I.a.208) only in that R¹ is =O:

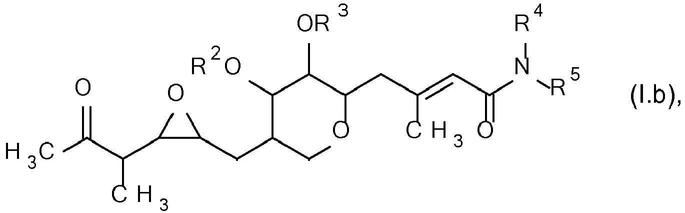

Also preferred are the amides of formula (I.c), particularly preferred the amides of formulae (I.c.1) to (I.c.208), which differ from the corresponding amides of formulae (I.a.1) to (I.a.208) only in that R¹ is O(CO)H:

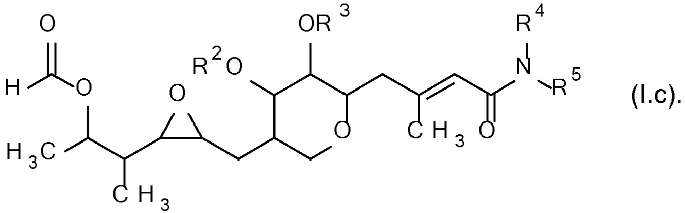

Also preferred are the amides of formula (I.d), particularly preferred the amides of formulae (I.d.1) to (I.d.208), which differ from the corresponding amides of formulae (I.a.1) to (I.a.208) only in that R¹ is O(CO)CH₃:

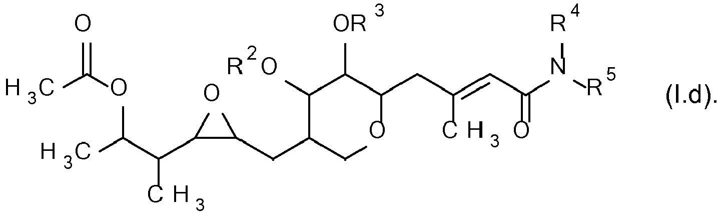

Also preferred are the amides of formula (I.1) (corresponds to formula (I) wherein the stereochemistry is defined as indicated in formula (I.1)): 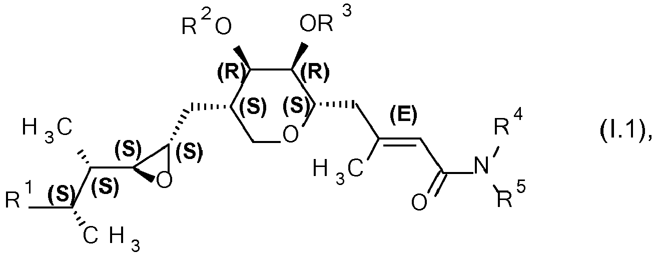 wherein the variables R¹, R², R³, R⁴ and R⁵ have the meanings, in particular the preferred meanings, as defined above.

Also preferred are the amides of formula (I.1.a), which correspond to formula (I.1) wherein R¹ is OH: 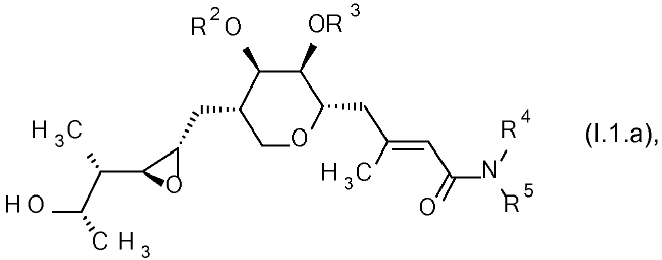 particularly preferred the amides of formulae (I.1.a.1) to (I.1.a,208), which differ from the corresponding amides of formulae (I.a.1) to (I.a.208) only in that the stereochemistry is defined as indicated in the formula (I.1).

Also preferred are the amides of formula (I.1.b), which correspond to formula (I.1) wherein R¹ is =O: 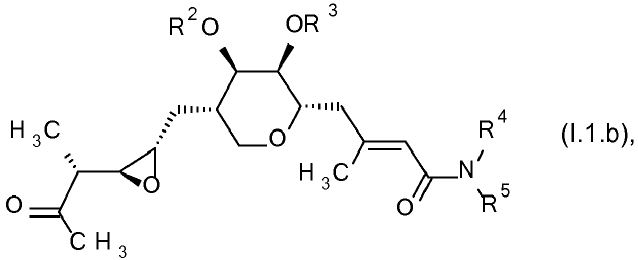 particularly preferred the amides of formulae (I.1.b.1) to (I.1.b.208), which differ from the corresponding amides of formulae (I.a.1) to (I.a.208) only in that R¹ is =O and that the stereochemistry is defined as indicated in the formula (I.1).

Also preferred are the amides of formula (I.1.c), which correspond to formula (I.1) wherein R¹ is =O(CO)H: 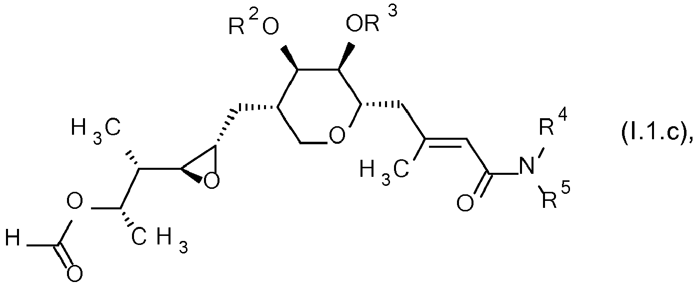 particularly preferred the amides of formulae (I.1.c.1) to (I.1.c.208), which differ from the corresponding amides of formulae (I.a.1) to (I.a.208) only in that R¹ is O(CO)H and that the stereochemistry is defined as indicated in the formula (I.1).

Also preferred are the amides of formula (I.1.d), which correspond to formula (I.1) wherein R¹ is =O(CO)CH₃: 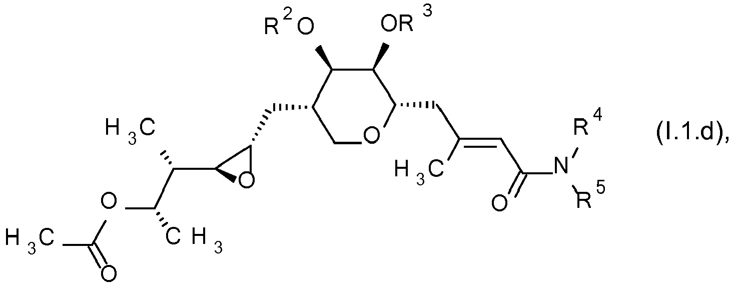 particularly preferred the amides of formulae (I.1.d.1) to (I.1.d.208), which differ from the corresponding amides of formulae (I.a.1) to (I.a.208) only in that R¹ is O(CO)CH₃ and that the stereochemistry is defined as indicated in the formula (I.1).

The amides of formula (I) according to the invention can be prepared by standard processes of organic chemistry, for example by the following process:

### Process A:

The amides of formula (I) are obtained by reaction of an acid of formula (III) (or its acetal protected form) with an amine of formula (II) using amide coupling conditions known from literature (e.g. WO 93/19599), as using a base and the corresponding amine in combination with an acid-activating reagent like HATU, carbodiimides, like dicyclohexylcarbodiimide, or a chlorcarbonate, like isobutylchlorcarbonate or ethylchlorcarbonate:

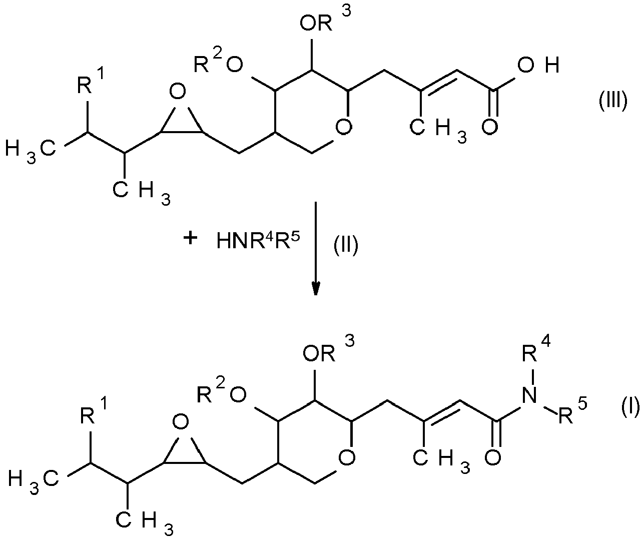

The conversion of the acid of formula (III) or salts thereof with an amine of formula (II) into the desired amide of formula (I) is carried out in the presence of an activating agent and, if appropriate, in the presence of a base, usually at temperatures of from 0°C to the boiling point of the reaction mixture, preferably from 0°C to 100°C, particularly preferably at room temperature, in an inert organic solvent [cf. Perich, J. W., Johns, R. B., J. Org. Chem. 53 (17), 4103-4105 (1988); Somlai, C. et al., Synthesis (3), 285-287 (1992); Gupta, A. et al., J. Chem. Soc. Perkin Trans. 2, 1911 (1990); Guan et al., J. Comb. Chem. 2, 297 (2000)].

Suitable activating agents are condensing agents, such as, for example, polystyrene-bound dicyclohexylcarbodiimide, diisopropylcarbodiimide, carbonyldiimidazole, chloroformic esters, such as methyl chloroformate, ethyl chloroformate, isopropyl chloroformate, isobutyl chloroformate, sec-butyl chloroformate or allyl chloroformate, pivaloyl chloride, polyphosphoric acid, propanephosphonic anhydride, bis(2-oxo-3-oxazolidinyl)phosphoryl chloride (BOPCI) or sulfonyl chlorides, such as methanesulfonyl chloride, toluenesulfonyl chloride or benzenesulfonyl chloride.

Suitable solvents are aliphatic hydrocarbons such as pentane, hexane, cyclohexane and mixtures of C₅-C₈-alkanes, aromatic hydrocarbons, such as benzene, toluene, o-, m- and p-xylene, halogenated hydrocarbons, such as methylene chloride, chloroform and chlorobenzene, ethers, such as diethyl ether, diisopropyl ether, tert-butyl methyl ether, dioxane, anisole and tetrahydrofuran (THF), nitriles, such as acetonitrile and propionitrile, ketones, such as acetone, methyl ethyl ketone, diethyl ketone and tert-butyl methyl ketone, alcohols, such as methanol, ethanol, n-propanol, isopropanol, n-butanol and tert-butanol, and also dimethyl sulfoxide, dimethylformamide (DMF), dimethylacetamide (DMA) and N-methylpyrrolidone (NMP), or else water; particular preference is given to methylene chloride, THF, methanol, ethanol and water.

It is also possible to use mixtures of the solvents mentioned.

Suitable bases are, in general, inorganic compounds, such as alkali metal and alkaline earth metal hydroxides, such as lithium hydroxide, sodium hydroxide, potassium hydroxide and calcium hydroxide, alkali metal and alkaline earth metal oxides, such as lithium oxide, sodium oxide, calcium oxide and magnesium oxide, alkali metal and alkaline earth metal hydrides, such as lithium hydride, sodium hydride, potassium hydride and calcium hydride, alkali metal and alkaline earth metal carbonates, such as lithium carbonate, potassium carbonate and calcium carbonate, and also alkali metal bicarbonates, such as sodium bicarbonate, moreover organic bases, for example tertiary amines, such as trimethylamine, triethylamine, diisopropylethylamine, N-methylmorpholine and N-methylpiperidine, pyridine, substituted pyridines, such as collidine, lutidine and 4-dimethylaminopyridine, and also bicyclic amines. Particular preference is given to sodium hydroxide, triethylamine, ethyl diisopropylamine, N-methylmorpholine and pyridine.

The bases are generally employed in catalytic amounts; however, they can also be employed in equimolar amounts, in excess or, if appropriate, as solvent.

The starting materials are generally reacted with one another in equimolar amounts. It may be advantageous to employ an excess of (II) based on (III).

Work-up and isolation of the products can be carried out in a manner known per se.

The amines of formula (II) required for preparing the amides of formula (I) are commercially available.

The acids of formula (III) required for preparing the amides of formula (I) are commercially available or can be prepared as described in literature.

Alternatively, the acids of formula (III) can also be directly generated from pseudomonic acid using a lipase enzyme, as described in WO 95/02064.

Specific amides of formula (I) can also be prepared as described in e.g. WO 93/19599, starting from pseudomonic acid (CAS 12650-69-0), which is available from several commercial sources. The 1,2-diol can be protected by cyclic acetals, like acetonide, formic acid ethyl ortho ester or benzaldehyde acetal. This can be achieved by reaction of the corresponding ketone, ketone dialkyl ketal, aldehyde, aldehyde dialkylacetal, alkoxypropene or trialkyl formic acid ortho ester under acid catalysis, e.g. with HCl, toluolsulfonic acid, acetic acid, sulfuric acid.

Alternatively, the hydroxy groups of pseudomonic acid can be protected as trialkylsilyl ethers, like triethylsilyl, trimethylsilyl, tert-butyl-dimethylsilyl, using the corresponding silylchlorides and a base, like triethylamine or pyridine.

Subsequently the ester can be cleaved, resulting in the alcohol protected monic acid derivatives. This ester cleavage can be achieved using a hydroxyde source, like lithium hydrixyde, sodium hydroxyde, potassium hydroxide.

This sequence can also be used to prepare free acid of formula (III) (e.g. monic acid, CAS 6262-68-8) without intermediate isolation of intermediates as described in US 4,237,161.

To widen the spectrum of action and to achieve synergistic effects, the amides of formula (I) may be combined with many representatives of other herbicidal or growth-regulating active ingredient groups and then applied concomitantly. Suitable components for combinations are, for example, herbicides from the classes of the acetamides, amides, aryloxyphenoxypropionates, benzamides, benzofuran, benzoic acids, benzothiadiazinones, bipyridylium, carbamates, chloroacetamides, chlorocarboxylic acids, cyclohexanediones, dinitroanilines, dinitrophenol, diphenyl ether, glycines, imidazolinones, isoxazoles, isoxazolidinones, nitriles, N-phenylphthalimides, oxadiazoles, oxazolidinediones, oxyacetamides, phenoxycarboxylic acids, phenyl-carbamates, phenylpyrazoles, phenylpyrazolines, phenylpyridazines, phosphinic acids, phosphoroamidates, phosphorodithioates, phthalamates, pyrazoles, pyridazinones, pyridines, pyridinecarboxylic acids, pyridinecarboxamides, pyrimidinediones, pyrimidinyl(thio)benzoates, quinolinecarboxylic acids, semicarbazones, sulfonylaminocarbonyltriazolinones, sulfonylureas, tetrazolinones, thiadiazoles, thiocarbamates, triazines, triazinones, triazoles, triazolinones, triazolocarboxamides, triazolopyrimidines, triketones, uracils, ureas.

It may furthermore be beneficial to apply the amides of formula (I) alone or in combination with other herbicides, or else in the form of a mixture with other crop protection agents, for example together with agents for controlling pests or phytopathogenic fungi or bacteria. Also of interest is the miscibility with mineral salt solutions, which are employed for treating nutritional and trace element deficiencies. Other additives such as non-phytotoxic oils and oil concentrates may also be added.

The invention also relates to formulations comprising at least an auxiliary and at least one amide of formula (I) according to the invention.

A formulation comprises a pesticidal effective amount of an amide of formula (I). The term "effective amount" denotes an amount of the amides of formula (I), which is sufficient for controlling undesired vegetation, especially for controlling undesired vegetation in crops (i.e. cultivated plants) and which does not result in a substantial damage to the treated crop plants. Such an amount can vary in a broad range and is dependent on various factors, such as the undesired vegetation to be controlled, the treated crop plants or material, the climatic conditions and the specific amides of formula (I) used.

The amides of formula (I) can be converted into customary types of formulations, e. g. solutions, emulsions, suspensions, dusts, powders, pastes, granules, pressings, capsules, and mixtures thereof. Examples for formulation types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). These and further formulation types are defined in the "Catalogue of pesticide formulation types and international coding system", Technical Monograph No. 2, 6th Ed. May 2008, CropLife International.

The formulations are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetting agents, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e.g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharides, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emulsifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-substituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-substituted fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinylpyrrolidone, vinylalcohols, or vinylacetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines or polyethyleneamines.

Suitable adjuvants are compounds, which have a neglectable or even no pesticidal activity themselves, and which improve the biological performance of the amides of formula (I) on the target. Examples are surfactants, mineral or vegetable oils, and other auxiliaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), inorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin.

Suitable anti-foaming agents are silicones, long chain alcohols, and salts of fatty acids.

Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants).

Suitable tackifiers or binders are polyvinylpyrrolidons, polyvinylacetates, polyvinyl alcohols, polyacrylates, biological or synthetic waxes, and cellulose ethers.

Examples for formulation types and their preparation are:

### i) Water-soluble concentrates (SL, LS)

10-60 wt% of an amide of formula (I) according to the invention and 5-15 wt% wetting agent (e.g. alcohol alkoxylates) are dissolved in water and/or in a water-soluble solvent (e.g. alcohols) ad 100 wt%. The active substance dissolves upon dilution with water.

### ii) Dispersible concentrates (DC)

5-25 wt% of an amide of formula (I) according to the invention and 1-10 wt% dispersant (e. g. polyvinylpyrrolidone) are dissolved in organic solvent (e.g. cyclohexanone) ad 100 wt%. Dilution with water gives a dispersion.

### iii) Emulsifiable concentrates (EC)

15-70 wt% of an amide of formula (I) according to the invention and 5-10 wt% emulsifiers (e.g. calcium dodecylbenzenesulfonate and castor oil ethoxylate) are dissolved in water-insoluble organic solvent (e.g. aromatic hydrocarbon) ad 100 wt%. Dilution with water gives an emulsion.

### iv) Emulsions (EW, EO, ES)

5-40 wt% of an amide of formula (I) according to the invention and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This mixture is introduced into water ad 100 wt% by means of an emulsifying machine and made into a homogeneous emulsion. Dilution with water gives an emulsion.

### v) Suspensions (SC, OD, FS)

In an agitated ball mill, 20-60 wt% of an amide of formula (I) according to the invention are comminuted with addition of 2-10 wt% dispersants and wetting agents (e.g. sodium lignosulfonate and alcohol ethoxylate), 0,1-2 wt% thickener (e.g. xanthan gum) and water ad 100 wt% to give a fine active substance suspension. Dilution with water gives a stable suspension of the active substance. For FS type formulation up to 40 wt% binder (e.g. polyvinylalcohol) is added.

### vi) Water-dispersible granules and water-soluble granules (WG, SG)

50-80 wt% of an amide of formula (I) according to the invention are ground finely with addition of dispersants and wetting agents (e.g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.

### vii) Water-dispersible powders and water-soluble powders (WP, SP, WS)

50-80 wt% of an amide of formula (I) according to the invention are ground in a rotor-stator mill with addition of 1-5 wt% dispersants (e.g. sodium lignosulfonate), 1-3 wt% wetting agents (e.g. alcohol ethoxylate) and solid carrier (e.g. silica gel) ad 100 wt%. Dilution with water gives a stable dispersion or solution of the active substance.

### viii) Gel (GW, GF)

In an agitated ball mill, 5-25 wt% of an amide of formula (I) according to the invention are comminuted with addition of 3-10 wt% dispersants (e.g. sodium lignosulfonate), 1-5 wt% thickener (e.g. carboxymethylcellulose) and water ad 100 wt% to give a fine suspension of the active substance. Dilution with water gives a stable suspension of the active substance.

### iv) Microemulsion (ME)

5-20 wt% of an amide of formula (I) according to the invention are added to 5-30 wt% organic solvent blend (e.g. fatty acid dimethylamide and cyclohexanone), 10-25 wt% surfactant blend (e.g. alcohol ethoxylate and arylphenol ethoxylate), and water ad 100 %. This mixture is stirred for 1 h to produce spontaneously a thermodynamically stable microemulsion.

### iv) Microcapsules (CS)

An oil phase comprising 5-50 wt% of an amide of formula (I) according to the invention, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 wt% of an amide of formula (I) according to the invention, 0-40 wt% water insoluble organic solvent (e.g. aromatic hydrocarbon), and an isocyanate monomer (e.g. diphenylmethene-4,4'-diisocyanate) are dispersed into an aqueous solution of a protective colloid (e.g. polyvinyl alcohol). The addition of a polyamine (e.g. hexamethylenediamine) results in the formation of polyurea microcapsules. The monomers amount to 1-10 wt%. The wt% relate to the total CS formulation.

### ix) Dustable powders (DP, DS)

1-10 wt% of an amide of formula (I) according to the invention are ground finely and mixed intimately with solid carrier (e.g. finely divided kaolin) ad 100 wt%.

### x) Granules (GR, FG)

0.5-30 wt% of an amide of formula (I) according to the invention is ground finely and associated with solid carrier (e.g. silicate) ad 100 wt%. Granulation is achieved by extrusion, spray-drying or the fluidized bed.

### xi) Ultra-low volume liquids (UL)

1-50 wt% of an amide of formula (I) according to the invention are dissolved in organic solvent (e.g. aromatic hydrocarbon) ad 100 wt%.

The formulation types i) to xi) may optionally comprise further auxiliaries, such as 0,1-1 wt% bactericides, 5-15 wt% anti-freezing agents, 0,1-1 wt% anti-foaming agents, and 0,1-1 wt% colorants.

The formulations comprising generally comprise between 0.01 and 95%, preferably between 0.1 and 90%, and in particular between 0.5 and 75%, by weight of the amides of formula (I). The amides of formula (I) are employed in a purity of from 90% to 100%, preferably from 95% to 100% (according to NMR spectrum).

Solutions for seed treatment (LS), suspoemulsions (SE), flowable concentrates (FS), powders for dry treatment (DS), water-dispersible powders for slurry treatment (WS), water-soluble powders (SS), emulsions (ES), emulsifiable concentrates (EC) and gels (GF) are usually employed for the purposes of treatment of plant propagation materials, particularly seeds. The formulations in question give, after two-to-tenfold dilution, active substance concentrations of from 0.01 to 60% by weight, preferably from 0.1 to 40% by weight, in the ready-to-use preparations.

Methods for applying amides of formula (I), or formulations thereof, on to plant propagation material, especially seeds, include dressing, coating, pelleting, dusting, soaking and in-furrow application methods of the propagation material. Preferably, amides of formula (I), or formulations thereof, are applied on to the plant propagation material by a method such that germination is not induced, e. g. by seed dressing, pelleting, coating and dusting.

Various types of oils, wetting agents, adjuvants, fertilizer, or micronutrients, and further pesticides (e.g. herbicides, growth regulators, safeners) may be added to the amides of formula (I), or the formulations thereof, as premix or, if appropriate not until immediately prior to use (tank mix). These agents can be admixed with the formulations according to the invention in a weight ratio of 1:100 to 100:1, preferably 1:10 to 10:1.

The user applies the amides of formula (I) according to the invention, or the formulations comprising them, usually from a pre-dosage device, a knapsack sprayer, a spray tank, a spray plane, or an irrigation system. Usually, the formulation is made up with water, buffer, and/or further auxiliaries to the desired application concentration and the ready-to-use spray liquor or the formulation according to the invention is thus obtained. Usually, 20 to 2000 liters, preferably 50 to 400 liters, of the ready-to-use spray liquor are applied per hectare of agricultural useful area.

According to one embodiment, either individual components of the formulation according to the invention or partially premixed components, e. g. components comprising amides of formula (I), may be mixed by the user in a spray tank and further auxiliaries and additives may be added, if appropriate.

In a further embodiment, individual components of the formulation according to the invention such as parts of a kit may be mixed by the user himself in a spray tank and further auxiliaries may be added, if appropriate.

In a further embodiment, either individual components of the formulation according to the invention or partially premixed components, e. g components comprising amides of formula (I), can be applied jointly (e.g. after tank mix) or consecutively.

The amides of formula (I) are suitable as herbicides. They are suitable as such or as an appropriate formulation.

The amides of formula (I) or the formulations comprising them control undesired vegetation on non-crop areas very efficiently, especially at high rates of application. They act against broadleaved weeds and grass weeds in crops such as wheat, rice, maize, soya and cotton without causing any significant damage to the crop plants. This effect is mainly observed at low rates of application.

The amides of formula (I) or the formulations comprising them are applied to the plants mainly by spraying the leaves. Here, the application can be carried out using, for example, water as carrier by customary spraying techniques using spray liquor amounts of from about 50 to 1000 l/ha (for example from 300 to 400 l/ha). The amides of formula (I) or the formulations comprising them may also be applied by the low-volume or the ultra-low-volume method, or in the form of microgranules.

Application of the amides of formula (I) or the formulations comprising them can be done before, during and/or after, preferably during and/or after, the emergence of the undesired vegetation. Application of the amides of formula (I) or the formulations comprising them can be carried out before or during sowing.

The amides of formula (I) or the formulations comprising them can be applied pre-, post-emergence or pre-plant, or together with the seed of a crop plant. It is also possible to apply the amides of formula (I), or the formulations comprising them, by applying seed, pretreated with the amides of formula (I), or the formulations comprising them, of a crop plant. If the active ingredients are less well tolerated by certain crop plants, application techniques may be used in which the amides of formula (I) or the formulations comprising them are sprayed, with the aid of the spraying equipment, in such a way that as far as possible they do not come into contact with the leaves of the sensitive crop plants, while the active ingredients reach the leaves of undesired vegetation growing underneath, or the bare soil surface (post-directed, lay-by).

In a further embodiment, the amides of formula (I), or the formulations comprising them, can be applied by treating seed. The treatment of seeds comprises essentially all procedures familiar to the person skilled in the art (seed dressing, seed coating, seed dusting, seed soaking, seed film coating, seed multilayer coating, seed encrusting, seed dripping and seed pelleting) based on the amides of formula (I) or the formulations comprising them.

The term "seed" comprises seed of all types, such as, for example, corns, seeds, fruits, tubers, seedlings and similar forms. Here, preferably, the term seed describes corns and seeds. The seed used can be seed of the crop plants mentioned above, but also the seed of transgenic plants or plants obtained by customary breeding methods.

When employed in plant protection, the amount of active substance applied, i.e. the amide of formula (I) without formulation auxiliaries, are, depending on the kind of effect desired, from 1 to 2000 g per ha, preferably from 5 to 2000 g per ha, more preferably from 20 to 900 g per ha and in particular from 20 to 200 g/ha, more preferred 20 to 100 g per ha.

In treatment of plant propagation materials such as seeds, e. g. by dusting, coating or drenching seed, amounts of active substance of from 0.1 to 1000 g, preferably from 1 to 1000 g, more preferably from 1 to 100 g and most preferably from 5 to 100 g, per 100 kilogram of plant propagation material (preferably seeds) are generally required.

In another embodiment of the invention, to treat the seed the amides of formula (I) are generally employed in amounts of from 0.001 to 10 kg per 100 kg of seed.

Depending on the application method in question, the amides of formula (I), or the formulations comprising them, can additionally be employed in a further number of crop plants for eliminating undesired vegetation.

Examples of suitable crops are the following:
Allium cepa, Ananas comosus, Arachis hypogaea, Asparagus officinalis, Avena sativa, Beta vulgaris spec. altissima, Beta vulgaris spec. rapa, Brassica napus var. napus, Brassica napus var. napobrassica, Brassica rapa var. silvestris, Brassica oleracea, Brassica nigra, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pistacia vera, Pisum sativum, Prunus avium, Prunus persica, Pyrus communis, Prunus armeniaca, Prunus cerasus, Prunus dulcis and Prunus domestica, Ribes sylvestre, Ricinus communis, Saccharum officinarum, Secale cereale, Sinapis alba, Solanum tuberosum, Sorghum bicolor (s. vulgare), Theobroma cacao, Trifolium pratense, Triticum aestivum, Triticale, Triticum durum, Vicia faba, Vitis vinifera and Zea mays.

Preferred crops are Arachis hypogaea, Beta vulgaris spec. altissima, Brassica napus var. napus, Brassica oleracea, Citrus limon, Citrus sinensis, Coffea arabica (Coffea canephora, Coffea liberica), Cynodon dactylon, Glycine max, Gossypium hirsutum, (Gossypium arboreum, Gossypium herbaceum, Gossypium vitifolium), Helianthus annuus, Hordeum vulgare, Juglans regia, Lens culinaris, Linum usitatissimum, Lycopersicon lycopersicum, Malus spec., Medicago sativa, Nicotiana tabacum (N.rustica), Olea europaea, Oryza sativa , Phaseolus lunatus, Phaseolus vulgaris, Pistacia vera, Pisum sativum, Prunus dulcis, Saccharum officinarum, Secale cereale, Solanum tuberosum, Sorghum bicolor (s. vulgare), Triticale, Triticum aestivum, Triticum durum, Vicia faba, Vitis vinifera and Zea mays.

Especially preferred crops are crops of cereals, corn, soybeans, rice, oilseed rape, cotton, potatoes, peanuts or permanent crops.

The amides of formula (I) according to the invention, or the formulations comprising them, can also be used in crops which have been modified by mutagenesis or genetic engineering in order to provide a new trait to a plant or to modify an already present trait.

The term "crops" as used herein includes also (crop) plants which have been modified by mutagenesis or genetic engineering in order to provide a new trait to a plant or to modify an already present trait.

Mutagenesis includes techniques of random mutagenesis using X-rays or mutagenic chemicals, but also techniques of targeted mutagenesis, in order to create mutations at a specific locus of a plant genome. Targeted mutagenesis techniques frequently use oligonucleotides or proteins like CRISPR/Cas, zinc-finger nucleases, TALENs or meganucleases to achieve the targeting effect.

Genetic engineering usually uses recombinant DNA techniques to create modifications in a plant genome which under natural circumstances cannot readily be obtained by cross breeding, mutagenesis or natural recombination. Typically, one or more genes are integrated into the genome of a plant to add a trait or improve a trait. These integrated genes are also referred to as transgenes in the art, while plant comprising such transgenes are referred to as transgenic plants. The process of plant transformation usually produces several transformation events, which differ in the genomic locus in which a transgene has been integrated. Plants comprising a specific transgene on a specific genomic locus are usually described as comprising a specific "event", which is referred to by a specific event name. Traits which have been introduced in plants or have been modified include in particular herbicide tolerance, insect resistance, increased yield and tolerance to abiotic conditions, like drought.

Herbicide tolerance has been created by using mutagenesis as well as using genetic engineering. Plants which have been rendered tolerant to acetolactate synthase (ALS) inhibitor herbicides by conventional methods of mutagenesis and breeding comprise plant varieties commercially available under the name Clearfield®. However, most of the herbicide tolerance traits have been created via the use of transgenes.

Herbicide tolerance has been created to glyphosate, glufosinate, 2,4-D, dicamba, oxynil herbicides, like bromoxynil and ioxynil, sulfonylurea herbicides, ALS inhibitor herbicides and 4-hydroxyphenylpyruvate dioxygenase (HPPD) inhibitors, like isoxaflutole and mesotrione.

Transgenes which have been used to provide herbicide tolerance traits comprise: for tolerance to glyphosate: cp4 epsps, epsps grg23ace5, mepsps, 2mepsps, gat4601, gat4621 and goxv247, for tolerance to glufosinate: pat and bar, for tolerance to 2,4-D: aad-1 and aad-12, for tolerance to dicamba: dmo, for tolerance to oxynil herbicies: bxn, for tolerance to sulfonylurea herbicides: zm-hra, csr1-2, gm-hra, S4-HrA, for tolerance to ALS inhibitor herbicides: csr1-2, for tolerance to HPPD inhibitor herbicides: hppdPF, W336 and avhppd-03.

Transgenic corn events comprising herbicide tolerance genes are for example, but not excluding others, DAS40278, MON801, MON802, MON809, MON810, MON832, MON87411, MON87419, MON87427, MON88017, MON89034, NK603, GA21, MZHG0JG, HCEM485, VCO-01981-5, 676, 678, 680, 33121, 4114, 59122, 98140, Bt10, Bt176, CBH-351, DBT418, DLL25, MS3, MS6, MZIR098, T25, TC1507 and TC6275.

Transgenic soybean events comprising herbicide tolerance genes are for example, but not excluding others, GTS 40-3-2, MON87705, MON87708, MON87712, MON87769, MON89788, A2704-12, A2704-21, A5547-127, A5547-35, DP356043, DAS44406-6, DAS68416-4, DAS-81419-2, GU262, SYHTØH2, W62, W98, FG72 and CV127.

Transgenic cotton events comprising herbicide tolerance genes are for example, but not excluding others, 19-51a, 31707, 42317, 81910, 281-24-236, 3006-210-23, BXN10211, BXN10215, BXN10222, BXN10224, MON1445, MON1698, MON88701, MON88913, GHB119, GHB614, LLCotton25, T303-3 and T304-40.

Transgenic canola events comprising herbicide tolerance genes are for example, but not excluding others, MON88302, HCR-1, HCN10, HCN28, HCN92, MS1, MS8, PHY14, PHY23, PHY35, PHY36, RF1, RF2 and RF3.

Insect resistance has mainly been created by transferring bacterial genes for insecticidal proteins to plants. Transgenes which have most frequently been used are toxin genes of *Bacillus spec.* and synthetic variants thereof, like cry1A, cry1Ab, cry1Ab-Ac, cry1Ac, cry1A.105, cry1F, cry1Fa2, cry2Ab2, cry2Ae, mcry3A, ecry3.1Ab, cry3Bb1, cry34Ab1, cry35Ab1, cry9C, vip3A(a), vip3Aa20. However, also genes of plant origin have been transferred to other plants. In particular genes coding for protease inhibitors, like CpTI and pinll. A further approach uses transgenes in order to produce double stranded RNA in plants to target and downregulate insect genes. An example for such a transgene is dvsnf7.

Transgenic corn events comprising genes for insecticidal proteins or double stranded RNA are for example, but not excluding others, Bt10, Bt11, Bt176, MON801, MON802, MON809, MON810, MON863, MON87411, MON88017, MON89034, 33121, 4114, 5307, 59122, TC1507, TC6275, CBH-351, MIR162, DBT418 and MZIR098.

Transgenic soybean events comprising genes for insecticidal proteins are for example, but not excluding others, MON87701, MON87751 and DAS-81419.

Transgenic cotton events comprising genes for insecticidal proteins are for example, but not excluding others, SGK321, MON531, MON757, MON1076, MON15985, 31707, 31803, 31807, 31808, 42317, BNLA-601, Event1, COT67B, COT102, T303-3, T304-40, GFM Cry1A, GK12, MLS 9124, 281-24-236, 3006-210-23, GHB119 and SGK321.

Increased yield has been created by increasing ear biomass using the transgene athb17, being present in corn event MON87403, or by enhancing photosynthesis using the transgene bbx32, being present in the soybean event MON87712.

Crops comprising a modified oil content have been created by using the transgenes: gm-fad2-1, Pj.D6D, Nc.Fad3, fad2-1A and fatb1-A. Soybean events comprising at least one of these genes are: 260-05, MON87705 and MON87769.

Tolerance to abiotic conditions, in particular to tolerance to drought, has been created by using the transgene cspB, comprised by the corn event MON87460 and by using the transgene Hahb-4, comprised by soybean event IND-ØØ41Ø-5.

Traits are frequently combined by combining genes in a transformation event or by combining different events during the breeding process. Preferred combination of traits are herbicide tolerance to different groups of herbicides, insect tolerance to different kind of insects, in particular tolerance to lepidopteran and coleopteran insects, herbicide tolerance with one or several types of insect resistance, herbicide tolerance with increased yield as well as a combination of herbicide tolerance and tolerance to abiotic conditions.

Plants comprising singular or stacked traits as well as the genes and events providing these traits are well known in the art. For example, detailed information as to the mutagenized or integrated genes and the respective events are available from websites of the organizations "International Service for the Acquisition of Agri-biotech Applications (ISAAA)" (http://www.isaaa.org/gmapprovaldatabase) and the "Center for Environmental Risk Assessment (CERA)" (http://cera-qmc.org/GMCropDatabase), as well as in patent applications, like EP3028573 and WO2017/011288.

The use of the amides of formula (I), or of formulations comprising them, according to the invention on crops may result in effects which are specific to a crop comprising a certain gene or event. These effects might involve changes in growth behavior or changed resistance to biotic or abiotic stress factors. Such effects may in particular comprise enhanced yield, enhanced resistance or tolerance to insects, nematodes, fungal, bacterial, mycoplasma, viral or viroid pathogens as well as early vigour, early or delayed ripening, cold or heat tolerance as well as changed amino acid or fatty acid spectrum or content.

Furthermore, plants are also covered that contain by the use of recombinant DNA techniques a modified amount of ingredients or new ingredients, specifically to improve raw material production, e.g., potatoes that produce increased amounts of amylopectin (e.g. Amflora® potato, BASF SE, Germany).

Furthermore, it has been found that the amides of formula (I) or the formulations comprising them, are also suitable for the defoliation and/or desiccation of plant parts of crops such as cotton, potato, oilseed rape, sunflower, soybean or field beans, in particular cotton. In this regard, formulations for the desiccation and/or defoliation of crop plants, processes for preparing these formulations, and methods for desiccating and/or defoliating plants using the amides of formula (I) have been found.

As desiccants, the amides of formula (I) are particularly suitable for desiccating the above-ground parts of crop plants such as potato, oilseed rape, sunflower and soybean, but also cereals. This makes possible the fully mechanical harvesting of these important crop plants. Also of economic interest is to facilitate harvesting, which is made possible by concentrating within a certain period of time the dehiscence, or reduction of adhesion to the tree, in citrus fruit, olives and other species and varieties of pernicious fruit, stone fruit and nuts. The same mechanism, i.e. the promotion of the development of abscission tissue between fruit part or leaf part and shoot part of the plants is also essential for the controlled defoliation of useful plants, in particular cotton.

Moreover, a shortening of the time interval in which the individual cotton plants mature leads to an increased fiber quality after harvesting.

The preparation of the amides of formula (I) is illustrated by examples; however, the subject matter of the present invention is not limited to the examples given.

The products shown below were characterized by the mass ([m/z]) or retention time (RT; [min.]) determined by HPLC-MS spectrometry.

HPLC-MS = high performance liquid chromatography-coupled mass spectrometry; HPLC column:
RP-18 column (Chromolith Speed ROD from Merck KgaA, Germany), 50*4.6 mm; mobile phase: acetonitrile + 0.1% trifluoroacetic acid (TFA)/water + 0.1% TFA using a gradient from 5:95 to 100:0 over 5 minutes at 40°C, flow rate 1.8 ml/min.

MS: quadrupole electrospray ionization, 80 V (positive mode).

The following abbreviations are used:
CH₂Cl₂: Dichloromethane
CH: Cyclohexane
DIEA: Diispropylethylamine
DMAP: 4-Dimethylaminopyridine
DMF: N,N-Dimethylformamide
EtOAc: Acetic acid ethyl ester
HATU:O-(7-Azabenzotriazol-1-yl)-N,N,N',N'-tetramethyluronium-hexafluorphosphate
HCl: hydrogen chloride
HPLC: High pressure chromatography
LC: Liquid chromatography
MeCN: Acetonitrile
MeOH: Methanol
MS: Mass spectrometry
MTBE: Methyl-tert-butylether
NaOH: Sodium hydroxyde
PE: Petrolether
THF: Tetrahydrofuran
TsOH*H2O: Toluolsulfonic acid hydrate

### A Preparation examples

### Example 1 (table 1 compound 26):

(E)-4-[(2S,3R,4R,5S)-3,4-dihydroxy-5-[[(2S,3S)-3-[(1S,2S)-2-hydroxy-1-methyl-propyl]oxiran-2-yl]methyl]tetrahydropyran-2-yl]-3-methyl-N-prop-2-ynyl-but-2-enamide

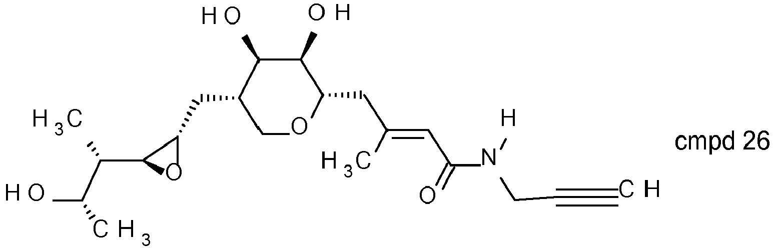

### Modified procedure from US 4,237,161:

To a solution of pseudomonic acid (CAS 12650-69-0; 20 g, 40 mmol) in HC(OEt)₃ (100 mL) was added TsOH*H2O (5mg) at 15°C. The mixture was stirred at 15°C for 12 h. The mixture was concentrated in vacuum to give the corresponding ortho-ester (crude in HC(OEt)₃). The crude product was used directly for the next step.

To a solution of the ortho-ester (crude in HC(OEt)₃) in H2O (400 mL) was added NaOH (16 g, 0.4 mol) in H2O at 0°C dropwise. The mixture was stirred at 65°C for 24 h under N₂. The mixture was adjusted to pH = 8 with 1N H₂SO₄ and concentrated to give the monic acid ortho ester (80 g, crude).

To a solution of the crude monic acid ortho ester (150 g, 300 mmol) was added propargylamine (33 g, 600 mmol), HATU (228 g, 600 mmol) and Na₂CO₃ (63.6 g, 600 mmol) in DMF (2 L) and stirred at 15°C for 12 h. The mixture was poured into ice water, extracted with EtOAc, washed with brine, dried, filtered and evaporated to give the corresponding amide (90 g, crude).

The solution of the crude amide (40 g, 74.2 mmol) in MeOH (400 mL) and H₂O (200 mL) was adjusted to pH = 2 with 1N HCl. The mixture was stirred at 0°C for 1h. Then the mixture was adjusted to pH = 9 with aqueous Na₂CO₃ and stirred for 3 h at 0°C and adjusted to pH=8 with 1N HCl. The resulting mixture was concentrated at 20°C to give the crude product. It was purified by preparative HPLC (neutral, MeCN-H₂O) to give (E)-4-[(2S,3R,4R,5S)-3,4-dihydroxy-5-[[(2S,3S)-3-[(1S,2S)-2-hydroxy-1-methyl-propyl]oxiran-2-yl]methyl]tetrahydropyran-2-yl]-3-methyl-N-prop-2-ynyl-but-2-enamide (10.1 g,35.7%) as a white solid.
¹HNMR (400MHz, methanol-d4): δ (ppm) 5.74 (s, 1H), 3.96 (d, J=2.5 Hz, 2H), 3.89 - 3.84 (m, 2H), 3.82 - 3.70 (m, 2H), 3.55 (dd, J=1.6, 11.5 Hz, 1H), 3.35 (dd, J=3.1, 9.0 Hz, 1H), 2.81 (dt, J=2.2, 5.8 Hz, 1H), 2.71 (dd, J=2.2, 7.6 Hz, 1H), 2.60 (br d, J=13.9 Hz, 1H), 2.55 (t, J=2.5 Hz, 1H), 2.20 - 2.13 (m, 4H), 1.96 (dt, J=4.1, 6.7 Hz, 1H), 1.68 (t, J=6.6 Hz, 2H), 1.44 - 1.37 (m, 1H), 1.20 (d, J=6.5 Hz, 3H), 0.95 (d, J=7.2 Hz, 3H)

### Example 2 (table 1 compound 35):

3-[(E)-4-[(2S,3R,4R,5S)-3,4-dihydroxy-5-[[(2S,3S)-3-[(1S,2S)-2-hydroxy-1-methyl-propyl]oxiran-2-yl]methyl]tetrahydropyran-2-yl]-3-methyl-but-2-enoyl]-5,5-dimethyl-oxazolidin-2-one

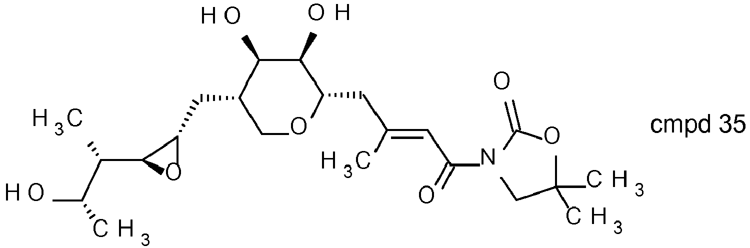

To a mixture of the crude monic acid ortho ester (procedure see above; 5 g, 10 mmol) in THF (150 mL) was added DMAP (1.22 g, 10 mmol) and HATU (11.4 g, 30 mmol) and stirred at 15°C for 48 h to give suspension A.

To a mixture of NaH (60%, 1.6 g, 40 mmol) in THF (150 mL) was added 5,5-dimethyloxazolidin-2-one (5.75 g, 50 mmol) and stirred at 60°C for 3 h to give suspension B.

The suspension B was added to suspension A and the resulting mixture was stirred at 15°C for 12 h. The mixture was poured into ice water, extracted with MTBE, washed with brine, dried, filtered and concentrated to give the crude amide (5 g, crude) as a yellow solid.

To a solution of crude amide (5 g, 8.3 mmol) in THF (50 mL) and H₂O (25 mL) was added 1N HCl adjust to pH=2. The mixture was stirred at 0°C for 1 h. Then the mixture was adjusted to pH=9 with aqueous Na2CO3 and stirred for 3 h at 0°C and adjusted to pH=8 with 1N HCl. The resulting mixture was concentrated at 15°C to give the crude product. The product was purified by preparative HPLC (neutral, MeCN-H₂O) to give 3-[(E)-4-[(2S,3R,4R,5S)-3,4-dihydroxy-5-[[(2S,3S)-3-[(1S,2S)-2-hydroxy-1-methyl-propyl]oxiran-2-yl]methyl]tetrahydropyran-2-yl]-3-ethyl-but-2-enoyl]-5,5-dimethyl-oxazolidin-2-one (450 mg, 12%) as a white solid.
¹H NMR (400 MHz, methanol-d4): δ (ppm)7.00 (s, 1H), 3.94 - 3.84 (m, 2H), 3.83 - 3.75 (m, 4H), 3.55 (br d, J=13.1 Hz, 1H), 3.40 (dd, J=3.0, 8.9 Hz, 1H), 2.81 (dt, J=2.2, 5.8 Hz, 1H), 2.74 - 2.66 (m, 2H), 2.34 - 2.26 (m, 1H), 2.19 (s, 3H), 1.96 (br d, J=3.4 Hz, 1H), 1.73 - 1.62 (m, 2H), 1.48 (s, 6H), 1.41 - 1.38 (m, 1H), 1.23 - 1.18 (m, 3H), 0.95 (d, J=7.2 Hz, 3H)

The compounds listed below in tables 1 to 3 can be prepared similarly to the examples mentioned above:

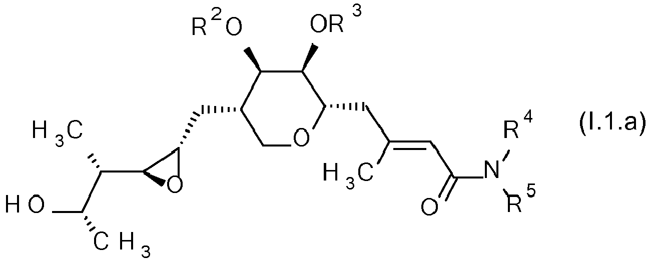

**Table 1**

| no. | R² | R³ | R⁴ | R⁵ | m/z [M+H] | Rₜ [min] |
|---|---|---|---|---|---|---|
| 1 | H | H | H | CH₂CH₂F | 389.9 | 0.664 |
| 2 | H | H | H | CH₂CHF₂ | 408.0 | 0.706 |
| 3 | H | H | H | CH₂CF₃ | 425.9 | 0.770 |
| 4 | H | H | H | CH₂CH₂CHF₂ | 422.0 | 0.732 |
| 5 | H | H | H | CH₂CH₂CF₃ | 440.0 | 0.788 |
| 6 | H | H | H | CH₂CF₂CH₃ | 422.0 | 0.748 |
| 7 | H | H | H | CH₂CF₂CHF₂ | 458.0 | 0.795 |
| 8 | H | H | H | CH₂CF₂CF₃ | 476.0 | 0.869 |
| 9 | H | H | H | CH₂CF₂CF₂CF₃ | 526.0 | 0.954 |
| 10 | (CO)H | H | H | CH₂CHF₂ | | |
| 11 | (CO)H | (CO)H | H | CH₂CHF₂ | 463.9 | 0.810 |
| 12 | (CO)H | (CO)H | H | CH₂CF₃ | 482.0 | 0.938 |
| 13 | -C(CH₃)₂- | | H | CH₂CH₂F | 430.1 | 0.956 |
| 14 | -C(CH₃)₂- | | H | CH₂CHF₂ | 448.1 | 0.959 |
| 15 | -C(CH₃)₂- | | H | CH₂CF₃ | 466.1 | 1.069 |
| 16 | -C(CH₃)₂- | | H | CH₂CF₂Br | 526 | 1.114 |
| 17 | -C(CH₃)₂- | | H | CH₂CF₂CH₃ | 462.2 | 1.020 |
| 18 | -C(CH₃)₂- | | H | *(S)*-CH(CH₃)(CF₃) | 480.1 | 1.117 |
| 19 | -C(CH₃)₂- | | H | CH₂CF₂CF₂CF₃ | 566 | 1.213 |
| 20 | -CH(OC₂H₅)- | | H | CH₂CF₃ | 482.0 | 0.834 |
| 21 | H | H | H | CH₂CH=CCl₂ | 451.9 | 0.859 |
| 22 | H | H | H | CH₂CF=CH₂ | 401.9 | 0.726 |
| 23 | -C(CH₃)₂- | | H | CH₂CF=CH₂ | 442.1 | 0.987 |
| 24 | -C(CH₃)₂- | | H | CH₂CCl=CH₂ | 458.1 | 1.027 |
| 25 | -C(CH₃)₂- | | H | CH₂CBr=CH₂ | 502.1 | 1.042 |
| 26 | H | H | H | CH₂C≡CH | 382.0 | 0.679 |
| 27 | H | H | H | CH₂C≡CCH₃ | 396.0 | 0.735 |
| 28 | H | H | H | CH(CH₃)C≡CH | 396.0 | 0.734 |
| 29 | H | H | H | CH₂C≡CC(CH₃)₃ | 438.1 | 0.931 |
| 30 | H | H | H | CH₂CH₂C≡CH | 396.0 | 0.709 |
| 31 | -C(CH₃)₂- | | H | CH₂C≡CH | 422.0 | 0.939 |
| 32 | -C(C₂H₅)₂- | | H | CH₂C≡CH | 450.1 | 1.068 |
| 33 | -CH(OC₂H₅)- | | H | CH₂C≡CH | 438.1 | 0.743 |
| 34 | H | H | -(CO)OCH₂CH₂- | | 414.0 | 0.716 |
| 35 | H | H | -(CO)OC(CH₃)₂CH₂- | | 442.0 | 0.833 |
| 36 | H | H | -(CO)OCH(CF₃)CH₂- | | 482.0 | 0.867 |
| 37 | H | H | -(CO)SCH₂CH₂- | | 429.9 | 0.798 |
| 38 | H | H | -(CO)N(CH₃)CH₂CH₂- | | 427.0 | 0.718 |
| 39 | -C(CH₃)₂- | | -(CO)SCH₂CH₂- | | 469.9 | 1.075 |
| 40 | -C(CH₃)₂- | | -(CO)N(CH₂CF₃)CH₂CH₂- | | 535.3 | 0.917 |
| 41 | -C(CH₃)₂- | | -(CO)N(CH₃)CH₂CH₂- | | 466.9 | 0.974 |

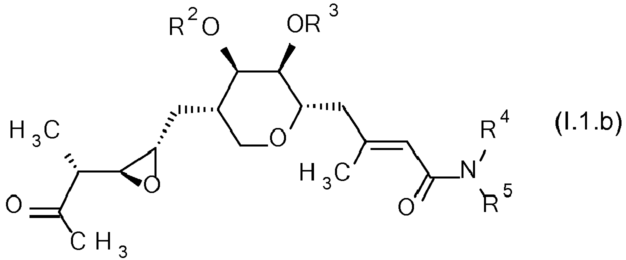

**Table 2**

| no. | R² | R³ | R⁴ | R⁵ | m/z [M+H] | Rₜ [min] |
|---|---|---|---|---|---|---|
| 42 | H | H | H | CH₂CHF₂ | 405.9 | 0.732 |
| 43 | H | H | H | CH₂CF₃ | 423.9 | 0.800 |
| 44 | (CO)H | (CO)H | H | CH₂CHF₂ | | |
| 45 | -C(CH₃)₂- | | H | CH₂CHF₂ | 445.0 | 1.018 |
| 46 | -C(CH₃)₂- | | H | CH₂CF₃ | 463.9 | 1.075 |
| 47 | (CO)CH₃ | (CO)CH₃ | H | CH₂C≡CH | 508.1 | 1.120 |
| 48 | -CH(OC₂H₅)- | | H | CH₂C≡CH | 436.1 | 0.793 |

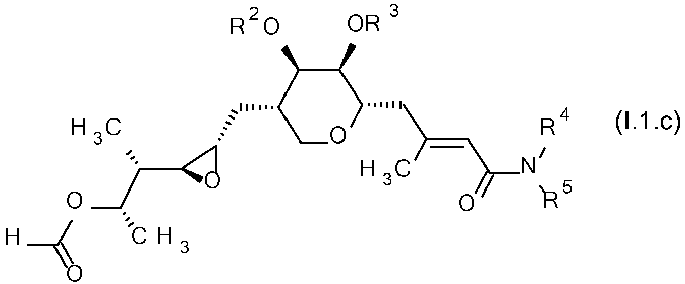

**Table 3**

| no. | R² | R³ | R⁴ | R⁵ | m/z [M+H] | Rₜ [min] |
|---|---|---|---|---|---|---|
| 49 | (CO)H | (CO)H | H | CH₂CHF₂ | 492.0 | 0.986 |
| 50 | (CO)H | (CO)H | H | CH₂CF₃ | 509.9 | 1.040 |

### B Use examples

The herbicidal activity of the amides of formula (I) was demonstrated by the following greenhouse experiments:
The culture containers used were plastic flowerpots containing loamy sand with approximately 3.0% of humus as the substrate. The seeds of the test plants were sown separately for each species.

For the pre-emergence treatment, the active ingredients, which had been suspended or emulsified in water, were applied directly after sowing by means of finely distributing nozzles. The containers were irrigated gently to promote germination and growth and subsequently covered with transparent plastic hoods until the plants had rooted. This cover caused uniform germination of the test plants, unless this had been impaired by the active ingredients.

For the post-emergence treatment, the test plants were first grown to a height of 3 to 15 cm, depending on the plant habit, and only then treated with the active ingredients which had been suspended or emulsified in water. For this purpose, the test plants were either sown directly and grown in the same containers, or they were first grown separately as seedlings and transplanted into the test containers a few days prior to treatment.

Depending on the species, the plants were kept at 10 - 25°C or 20 - 35°C, respectively.

The test period extended over 2 to 4 weeks. During this time, the plants were tended, and their response to the individual treatments was evaluated.

Evaluation was carried out using a scale from 0 to 100. 100 means no emergence of the plants, or complete destruction of at least the aerial moieties, and 0 means no damage, or normal course of growth. A good herbicidal activity is given at values of at least 80 and a very good herbicidal activity is given at values of at least 90.

The plants used in the greenhouse experiments were of the following species:

| Bayer code | Scientific name |
|---|---|
| ABUTH | Abutilon theophrasti |
| ALOMY | Alopercurus myosuroides |
| AMARE | Amaranthus retroflexus |
| AVEFA | Avena fatua |
| ECHCG | Echinocloa crus-galli |
| SETFA | Setaria faberi |
| SETVI | Setaria viridis |

At an application rate of 500 g/ha, compound 29 applied by the pre-emergence method, showed good herbicidal activity against SETFA.

At an application rate of 500 g/ha, compounds 8, 9 and 27 applied by the pre-emergence method, showed good herbicidal activity against ECHCG.

At an application rate of 500 g/ha, compound 33 applied by the pre-emergence method, showed good herbicidal activity against AMARE.

At an application rate of 500 g/ha, compounds 34 and 43 applied by the pre-emergence method, showed very good herbicidal activity against ECHCG.

At an application rate of 500 g/ha, compounds 1, 3, 4, 5, 6, 7, 8, 12, 13, 14, 15, 16, 17, 18, 22, 23, 24, 25, 26, 27, 31, 32, 33, 34, 35, 38, 50, 45, 46, 47 and 48 applied by the post-emergence method, showed very good herbicidal activity against ECHCG, SETVI, ABUTH and AMARE.

At an application rate of 500 g/ha, compounds 2, 21, 28, 29 and 40 applied by the post-emergence method, showed very good herbicidal activity against SETVI, ABUTH and AMARE, and good herbicidal activity against ECHCG.

At an application rate of 500 g/ha, compounds 11 and 49 applied by the post-emergence method, showed very good herbicidal activity against SETVI, ABUTH and AMARE, and good herbicidal activity against ALOMY.

At an application rate of 500 g/ha, the compounds 36 and 41 applied by the post-emergence method, showed very good herbicidal activity against SETVI, ABUTH and AMARE.

At an application rate of 500 g/ha, compound 9 applied by the post-emergence method, showed very good herbicidal activity against SETVI and AMARE, and good herbicidal activity against ECHCG.

At an application rate of 500 g/ha, compound 19 applied by the post-emergence method, showed very good herbicidal activity against AMARE.

At an application rate of 500 g/ha, compound 20 applied by the post-emergence method, showed very good herbicidal activity against ECHCG, ABUTH and AMARE.

At an application rate of 500 g/ha, compound 37 applied by the post-emergence method, showed very good herbicidal activity against SETVI and ABUTH, and good herbicidal activity against ECHCG and AMARE.

At an application rate of 500 g/ha, compound 39 applied by the post-emergence method, showed very good herbicidal activity against SETVI and ABUTH.

At an application rate of 500 g/ha, compound 43 applied by the post-emergence method, showed very good herbicidal activity against SETVI, ABUTH and AVEFA.

## Claims

1. Amides of formula (I) 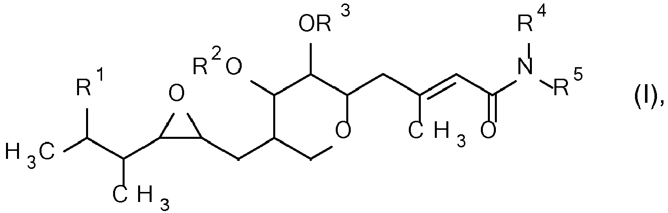 wherein the variables have the following meanings:
R¹ OH, =O or O(CO)R⁶,
wherein R⁶ is H or C₁-C₆-alkyl;
R² H or (CO)R⁷;
wherein R⁷ is H or C₁-C₆-alkyl;
R³ H or (CO)R⁸;
wherein R⁸ is H or C₁-C₆-alkyl; or
R² and R³ together form -CR⁹R¹⁰-,
wherein R⁹ and R¹⁰ independently of one another are H, C₁-C₆-alkyl or C₁-C₆-alkoxy;
R⁴ H;
R⁵ C₂-C₆-haloalkyl, C₃-C₆-haloalkenyl, C₃-C₆-alkynyl or C₃-C₆-haloalkynyl; or
R⁴ and R⁵ together form a 5- to 6-membered saturated heterocycle,
which is substituted with one carbonyl group, optionally has in addition to the N-atom one ring member selected from the group consisting of -N-, -O- and -S-, and
optionally is substituted with one or two substituents selected from C₁-C₃-alkyl and C₁-C₃-haloalkyl.

2. Amides of formula (I) according to claim 1, wherein R¹ is OH or =O.

3. Amides of formula (I) according to claim 1 or 2, wherein R¹ is OH.

4. Amides of formula (I) according to any of claims 1 to 3, wherein R² and R³ are independently of one another H, (CO)H or (CO)CH₃, or together form -CR⁹R¹⁰-,
wherein R⁹ is C₁-C₆-alkyl or C₁-C₆-alkoxy; and
R¹⁰ is H or C₁-C₆-alkyl.

5. Amides of formula (I) according to any of claims 1 to 4, wherein R⁵ is C₂-C₆-haloalkyl, C₃-C₆-haloalkenyl or C₃-C₆-alkynyl, or R⁴ and R⁵ together form a 5-membered saturated heterocycle, which is substituted with one carbonyl group,
optionally has in addition to the N-atom one ring member selected from the group consisting of -N-, -O- and -S-, and
optionally is substituted with one or two substituents selected from C₁-C₃-alkyl and C₁-C₃-haloalkyl.

6. Amides of formula (I) according to claim 1, wherein the amide is the amide of formula (1.1) 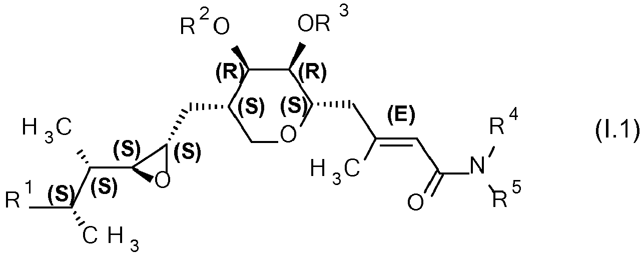 wherein the variables R¹, R², R³, R⁴ and R⁵ are as defined in claim 1.

7. Process for the preparation of amides of formula (I) as defined in claim 1, wherein an acid of formula (III) 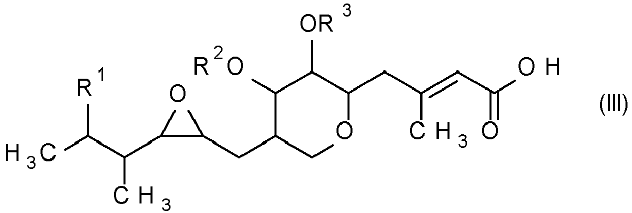 wherein R¹, R² and R³ are as defined in claim 1;
are reacted with an amine of formula (II)
HNR⁴R⁵ (II)
wherein R⁴ and R⁵ are as defined in claim 1.

8. A herbicidal composition comprising an herbicidally active amount of at least one amide of formula (I) as claimed in claim 1 and at least one inert liquid and/or solid carrier and, if appropriate, at least one surface-active substance.

9. A process for the preparation of herbicidal compositions, which comprises mixing an herbicidally active amount of at least one amide of formula (I) as claimed in claim 1 and at least one inert liquid and/or solid carrier and, if desired, at least one surface-active substance.

10. A method of controlling undesired vegetation, which comprises allowing an herbicidally active amount of at least one amide of formula (I) as claimed in claim 1 to act on plants, their environment or on seed.

11. The use of amides of formula (I) as claimed in claim 1 as herbicides.
